# EUROPEAN PATENT APPLICATION

(11) **EP 1 145 703 A1**
(43) Date of publication of application: **17.10.2001**
(21) Application number: 99961366.4
(22) Date of filing: 24.12.1999
(51) Int. Cl.: A61J 3/00, A61M 5/14

(54) **MEDICINE CONTAINER AND SYRINGE**

(30) Priority: 25.12.1998 JP 37119198; 25.12.1998 JP 37119298
(71) Applicant: Torii Pharmaceutical Co., Ltd., Chuo-ku, Tokyo 103-0023 (JP)
(72) Inventor: OKUTOME, Toshiyuki, Tokyo 158-0086 (JP); SUGIYAMA, Satoshi, Chiba 264-0026 (JP); KASHIWAGI, Takashi, Tokyo 134-0087 (JP); MEGURO, Toru, Chiba 260-0034 (JP); NATSUI, Kensuke, Saitama 343-0027 (JP)
(74) Representative: Bardo, Julian Eason
(86) International application number: JP9907277
(87) International publication number: WO0038615

(57) **Abstract**

By having a chamber for containing a medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, and breaking at least a portion of the breakable part to separate the closing part from a container, the medicine is supplied or injected to be contained in the container for medicine from which the medicine is discharged to the outside of the container through the broken breakable portion while the medicine is prevented from passing the inlet path.

## Description

### FIELD OF THE INVENTION

The present invention relates to a container for containing therein a medicine, and a syringe for containing therein the medicine.

### PRIOR ART

In an extracorporeal circulation path such as an artificial dialyser, a blood plasma separator, a blood circuit or the like, since a blood coagulation component is activated by a contact between a blood and an air bubble and/or between the blood and a substance other than an intima of blood vessel, a stagnation of blood flow or the like, it is necessary for a medicine for preventing the blood from coagulating to be supplied. An example of such medicine is nafamostat mesilate (protease inhibitor). In an operating sequence for setting the medicine on an apparatus, a dissolution liquid (for example, 5%-glucose injection) is drawn into a syringe by suction at first, and the dissolution liquid is injected from the syringe to the freeze-dried medicine contained by a glass container so that the medicine is dissolved in the dissolution liquid in the glass container to form a solution including the medicine. Subsequently, the solution including the medicine (for example, medicine total amount 10-300 mg) is drawn into the syringe by suction, and this is set on a syringe mounting portion of a dialyser (or syringe pump and so forth).

### SUMMARY OF THE INVENTION

An object of the present invention is to provide a medicine container by which a medicine contained by the medicine container is prevented effectively from being contaminated by an environment surrounding the container.

A container for a medicine, according to the invention, comprises, a chamber for containing the medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, wherein by breaking at least a portion of the breakable part to separate the closing part from the container, the medicine is allowed to be discharged to the outside of the container through the broken breakable part while the medicine is prevented from passing the inlet passage.

According to the invention, since the container for the medicine comprises the breakable part between the body and the closing part, and by breaking the at least a portion of the breakable part to separate the closing part from the container, the medicine is allowed to be discharged to the outside of the container through the broken breakable part while the medicine is prevented from passing the inlet passage, the medicine is taken out of the container through an opening newly formed by the broken breakable part so that taking the medicine out of the container through the extremely clean opening which has not been contaminated by an environment surrounding the container just after forming the opening becomes possible.

It is effective for preventing the contamination caused by an assembly that the body, the closing part and the breakable part are monolithic, or particularly that the body, the closing part and the breakable part are monolithically formed by a synthetic resin.

If a cross-sectional area of the breakable part along an imaginary plane perpendicular to a flow direction of the medicine discharged to the outside of the container is significantly smaller than a cross-sectional area of the body along the imaginary plane perpendicular to the flow direction of the medicine discharged to the outside of the container and a cross-sectional area of the closing part along the imaginary plane perpendicular to the flow direction of the medicine discharged to the outside of the container, an area of the breakable part newly exposed by breaking the breakable part is significantly small to minimize a deformation of the breakable part caused by breaking the breakable part.

If the container further comprises a stopper slideable in the inlet passage while sealing hermetically the inlet passage, a hermetic seal of the chamber is sufficient and a flow-out of the medicine to the outside of the container through the inlet passage is more securely prevented. The chamber may contain the medicine of dry condition.

If the container further comprises the stopper guided by the inlet passage to slide therein, and the inlet passage includes a first area which is relatively far away from the breakable part and forms a clearance between the inlet passage and the stopper to allow a gaseous communication between the chamber and the outside of the container, and a second area which is relatively near by the breakable part and is hermetically sealed by the stopper, both of freeze-dry of the medicine and the hermetic seal of the chamber containing the medicine are performed while the stopper is held in the inlet passage. If the container further comprises the stopper slideable in the inlet passage while sealing hermetically the inlet passage, and an injection needle, the medicine may be discharged to the outside of the container through the breakable part and the injection needle when the stopper moves in the inlet passage toward the breakable part. When the container further comprises a flange extending perpendicularly to a longitudinal axis of the inlet passage, a distance between the flange and the breakable part in a longitudinal direction of the inlet passage may be prevented from becoming not less than a predetermined distance. When the body includes a protrusion extending perpendicularly to a longitudinal axis of the inlet passage, the flange discrete relative to the body may contact the protrusion to prevent the distance between the flange and the breakable part in the longitudinal direction of the inlet passage from becoming not less than the predetermined distance.

If a cover covering the closing part extends over the breakable part to fit onto the body, the breakable part is prevented from being broken and a contamination of the breakable part is prevented. If the cover is prevented from contacting the breakable part when fitting onto the body, an undesirable damage of the breakable part is restrained.

A method for containing a medicine into a container, according to the invention, comprises the steps of:
preparing the medicine container comprising a chamber for containing the medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, wherein-the medicine is allowed to be discharged to the outside of the container through the broken breakable part when at least a portion of the breakable part is broken to separate the closing part from the container,
feeding the medicine into the chamber,
positioning a stopper at a first area of the inlet passage relatively far away from the breakable part so that a clearance between the inlet passage and the stopper is formed to allow a gaseous communication between the chamber and the outside of the container,
drying the medicine by discharging a moisture from the chamber through the clearance to the outside of the container, and
positioning the stopper at a second area of the inlet passage relatively near by the breakable part so that the inlet passage is hermetically sealed by the stopper.

According to the invention, since the container comprises the breakable part between the body and the closing part, and by breaking the at least a portion of the breakable part to separate the closing part from the container, the medicine is allowed to be discharged to the outside of the container through the broken breakable part while the medicine is prevented from passing the inlet passage, the medicine is taken out of the container through an opening newly formed by the broken breakable part so that taking the medicine out of the container through the extremely clean opening which has not been contaminated by an environment surrounding the container just after forming the opening becomes possible.

It is preferable for preventing an inside of the chamber from being contaminated and inserting securely the stopper into the inlet passage that a gaseous pressure in the chamber is less than an atmospheric pressure when the stopper is being positioned at the second area relatively near by the breakable part, and the container is arranged in a gaseous environment of the atmospheric pressure after the stopper is positioned at the second area relatively near by the breakable part. It is preferable for a smooth movement and sealing of the stopper in the inlet passage with a surface of the stopper formed of, for example, isobutylene-isoprene rubber and contacting the inlet passage that a temperature in the chamber is less than 60°C, preferably less than 50°C (preferably not less than 1°C) when the stopper is being positioned at the second area relatively near by the breakable part, and the container is arranged in an environment of room temperature (1-30°C) after the stopper is positioned at the second area relatively near by the breakable part.

It is effective for a supply of the medicine toward the closing part and a smooth movement of the medicine toward the opening formed by breaking the at least a portion of the breakable part that the inlet passage, closing part and breakable part are substantially aligned along an axis parallel to a longitudinal axis of the inlet passage.

In a medicine package set of the invention, a medicine of dry condition is contained by an inner side of a front end of a plastic syringe outer tube, and hermetically sealed by an elastic stopper detachably mounted on a front end of a plunger. At the front end of the syringe outer tube, a projection for a discharge hole with a ring-shaped cut-in groove for breaking is monolithically formed with the syringe outer tube, and a breaking tool operating also as a protecting cap may be fitted onto an outer surface of the projection for the discharge hole. Or a clearance forming recess for forming a clearance between the elastic stopper and a base portion of the syringe outer tube when the elastic stopper is fitted into the base portion of the syringe outer tube by a small depth may be formed on an inner surface of the base portion of the syringe outer tube. Further, the medicine package set may be hermetically sealed and contained by a hermetically sealing case made of an aluminum deposited film, silica deposited film or the like.

The syringe of the invention has a charged portion to be charged with the medicine, and a discharge portion forming a discharge passage through which the medicine is discharged from the charged portion, and the discharge portion is formed in such a manner that the discharge passage is closed, and has a weak part for releasing the closing of the discharge passage. In this structure, the medicine is prevented from being discharged to the outside when the discharge passage is closed, although the medicine in the charged portion is finally discharged from the discharge passage of the discharge portion. Therefore, it is possible that the medicine is inserted and the syringe charged with the medicine is set on a freeze-dry device when the discharge passage is closed. The closing of the discharge passage is released by breaking the weak part and the syringe is mounted on, for example, a syringe pump, when the medicine is actually used.

A mounting portion for a jig for breaking the weak part is preferably formed. The breaking means a release of the closing of the discharge passage by bending, twisting, cutting or the like. The weak part causes the release of the closing of the discharge passage with a small breaking force. The jig makes the breaking of the weak part easy. Therefore, in the above points, the syringe can be used easily.

In an embodiment of the invention, the weak part is formed at the front end of the discharge passage or adjacent to the front end, and it is preferable that the jig mounting portion on which the jig is mounted is formed adjacent to the weak part. In this structure, the discharge passage, weak part and jig mounting portion can be formed monolithically so that a producing cost of the syringe is advantageously decreased. Further, since the weak part and jig mounting portion are adjacent to each other, a breaking force by the jig is effectively applied to the weak part. A concrete shape of the jig mounting portion is, for example, a column-shaped body of rectangular cross section. If the jig to be mounted on the jig mounting portion with such shape has a column-shaped hole of rectangular cross section, the breaking force is easily applied to the weak part by twisting the jig. In another preferred embodiment of the invention, the discharge portion is formed at an end of the tube-shaped charged portion, a flange is formed at another end of the tube-shaped charged portion, and the flange and the charged portion are separable from each other. The flange is necessary for positioning the syringe when being mounted on the syringe pump, but is not convenient when the syringe charged with the medicine is mounted on the freeze-dry device. That is, although it is preferable that the syringes as many as possible are set vertically when being mounted on the freeze-dry device, the flange integrally arranged on the syringe decreases a space for setting the syringe. Therefore, the flange and the charged portion are separable from each other, the flange is detached when the syringe is mounted on the freeze-dry device, and the flange is attached when the syringe is set on the syringe pump. By this operation, the syringe is used easily.

### BRIEF DESCRIPTION OF THE DRAWINGS

Fig. 1 is a cross sectional view showing an embodiment of medicine package set of the invention.
Fig. 2 is a side view of a syringe outer tube.
Fig. 3 is a back view of the syringe outer tube.
Fig. 4 is an enlarged cross sectional view of a projection for a discharge hole.
Fig. 5A is a front view of an external flange.
Fig. 5B is a side view of the external flange.
Fig. 5C is a back view of the external flange.
Fig. 6 is a partially cross sectional view of an elastic stopper.
Fig. 7 is an oblique projection view of a cutting tool.
Fig. 8 is an oblique projection view of a disassembled syringe.
Fig. 9 is a cross sectional view showing an aligned condition in which the syringe outer tubes are charged with a medicine, and the elastic stoppers are fitted into by a small depth in a freeze-dry room.
Fig. 10 is an enlarged longitudinally-cross-sectional view showing a fitting condition between the syringe and the elastic stopper in the freeze-dry room.
Fig. 11 is an enlarged cross-sectional view showing the fitting condition between the syringe and the elastic stopper in the freeze-dry room.
Fig. 12 is an oblique projection view of a support member of the syringe outer tube.
Fig. 13 is an explanatory view of a device for further pressing downward the elastic stopper fitted by the small depth into the syringe outer tube after the freeze-dry in the freeze-dry room.
Fig. 14 is a cross sectional view showing a condition in which the freeze-dried medicine is hermetically sealed in the syringe outer tube by the elastic stopper.
Fig. 15 is an oblique projection view showing a sealing bag.
Fig. 16 is an oblique projection view showing a condition in which the sealing bag is cut to be opened.
Fig. 17 is an oblique projection view showing a condition in which the cutting tool is applied to the projection for the discharge hole of the syringe outer tube.
Fig. 18 is a plan view showing a condition in which the cutting tool is fitted onto a base portion of the projection for the discharge hole of the syringe outer tube.
Fig. 19 is a side view showing a condition in which the external flange is mounted on the base portion of the portion of the syringe outer tube, and an injection needle is applied to the base portion of the opened projection for the discharge hole.'
Fig. 20 is a partially cross sectional view showing a fitting between the syringe outer tube and the external flange as shown in Fig. 19.
Fig. 21 is an explanatory view of an operation for drawing a dissolution liquid into the syringe outer tube with suction.
Fig. 22 is an oblique projection view showing a condition in which the syringe is set onto a syringe mounting portion of a dialyser.
Fig. 23 is a plan view of the syringe mounting portion on which the syringe is mounted.
Fig. 24 is a circuit diagram showing an external circulation path.
Fig. 25 is a cross sectional view showing an entire structure of a syringe of another embodiment of the invention.
Fig. 26 is an enlarged cross sectional view of a discharge portion of the syringe as shown in Fig. 25.
Fig. 27 is a view of the syringe as seen from an arrow 10S in Fig. 25.
Fig. 28A is a first side view showing a jig (cover) to be mounted onto a jig (cover) mounting portion.
Fig. 28B is a partially cross sectional front view of the jig.
Fig. 28C is a second side view showing the jig.
Fig. 29 is a view showing the syringe and a detachable flange as seen from an arrow 10T in Fig. 25.
Fig. 30 is a cross sectional view showing an engaging condition between the syringe and the detachable flange.
Fig. 31A is a cross sectional view showing the syringe during the freeze-drying.
Fig. 31B is a cross sectional view showing the syringe after the freeze-drying
Fig. 32A is a partially cross sectional view showing an alternative embodiment of the flange at the end of the container of the invention.
Fig. 32B is a side view showing the alternative embodiment of the flange at the end of the container of the invention.
Fig. 33 is a view of the flange as shown in Figs. 32A and 32B as seen from an arrow in Fig. 32B.
Fig. 34 is an oblique projection schematic view of the flange as shown in Figs. 32A and 32B.

### DETAILED DESCRIPTION OF PREFERRED EMBODIMENTS OF THE INVENTION

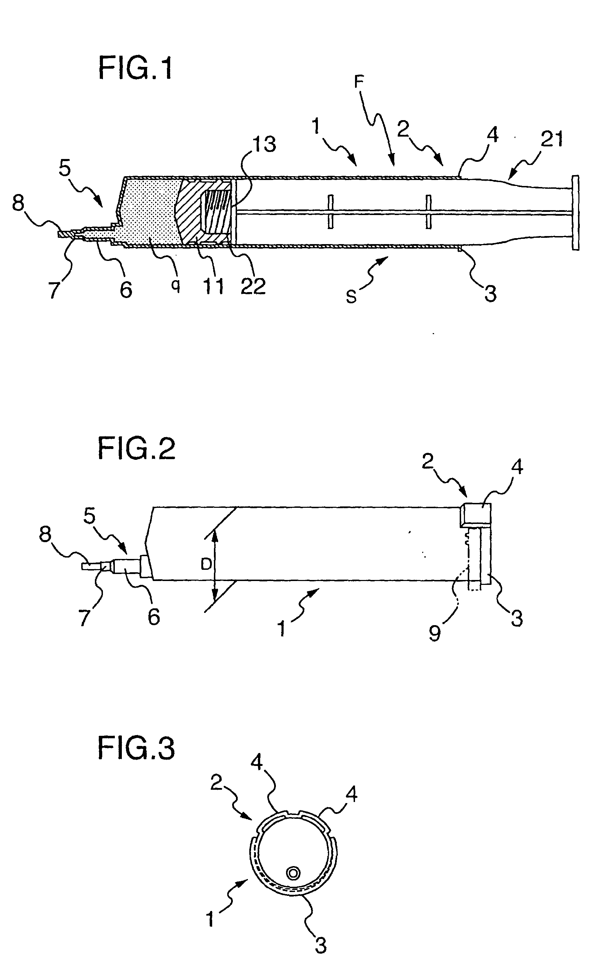

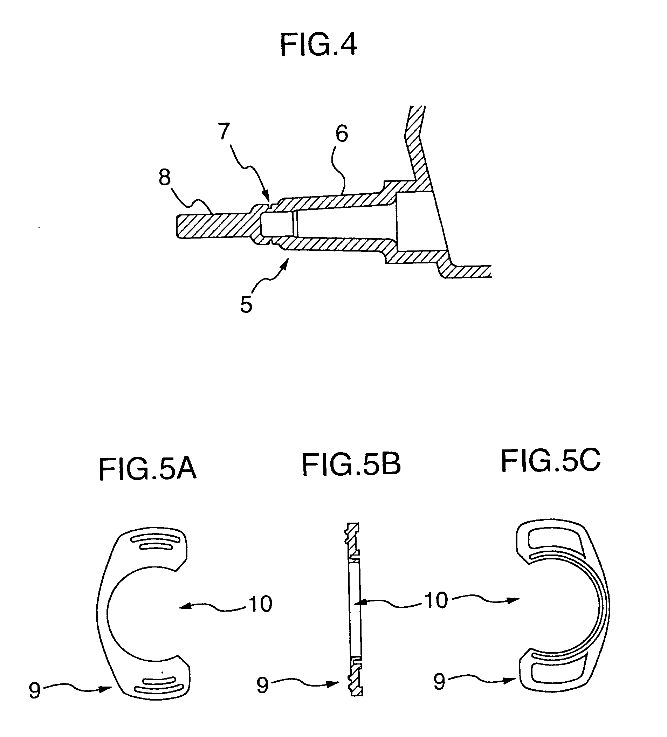

Fig. 1 is a cross sectional view of a medicine package set F made of a plastic material, and an engage flange 3 and two clearance forming recesses 4 opening outward are formed on a base portion of a syringe outer tube 1 (refer to Figs. 2 and 3). On the other hand, a tapered projection 5 for a discharge hole to be opened by being cut is formed at an outward front end of the syringe outer tube 1, and a cut portion 8 to be cut off is monolithically formed on a base portion 6 thereof through a ring-shaped cut groove 7 for being cut (hereafter, called as a cut groove) (refer to Fig. 4). A medicine q of dry condition by a freeze-dry is contained at an inside of the front end thereof with hermetic seal by an elastic stopper 11 contacting the syringe outer tube 1 through an elastic member such as rubber member or the like, and a plunger 21 is joined with the elastic stopper 11. Incidentally, the medicine is the above described nafamostat mesilate (protease inhibitor) as a general principle in the invention.

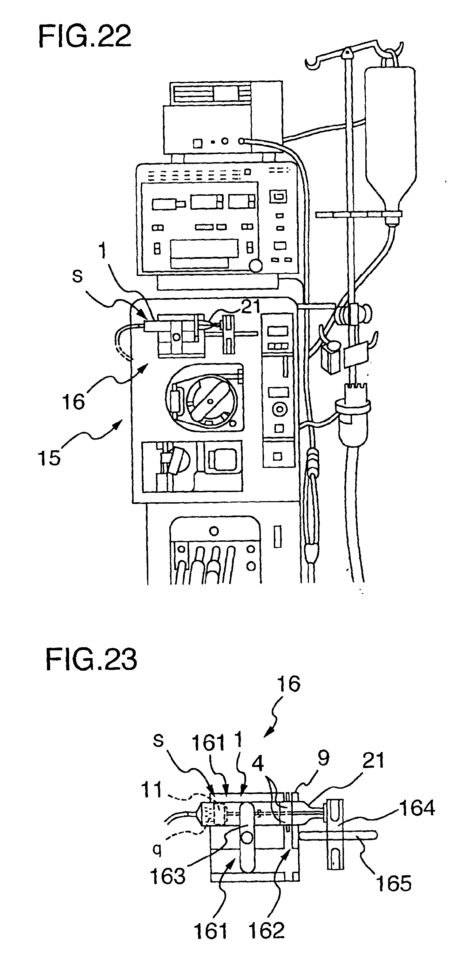

Figs. 5A-5C show an external flange 9, an opening 10 thereof is slightly smaller than an outer diameter D (refer to Fig. 2) of the syringe outer tube 1, and the external flange 9 is fitted detachably on a base portion 2 of the syringe outer tube 1 so that the external flange 9 is engaged with an engage groove 162 to be fixed when the syringe outer tube 1 is set on a syringe mounting portion 16 (refer to Figs. 22 and 23) of a dialyser (or syringe pump) 15 as described below.

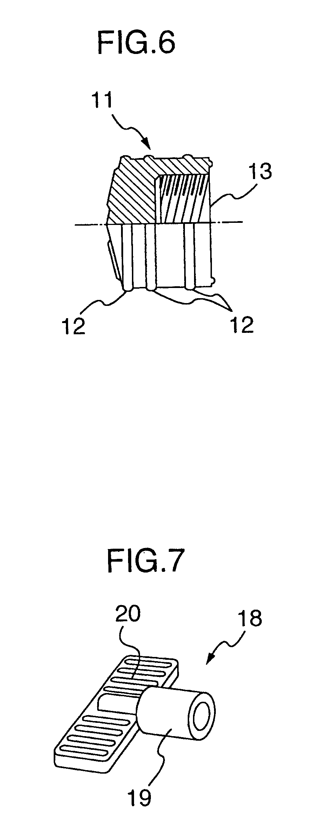

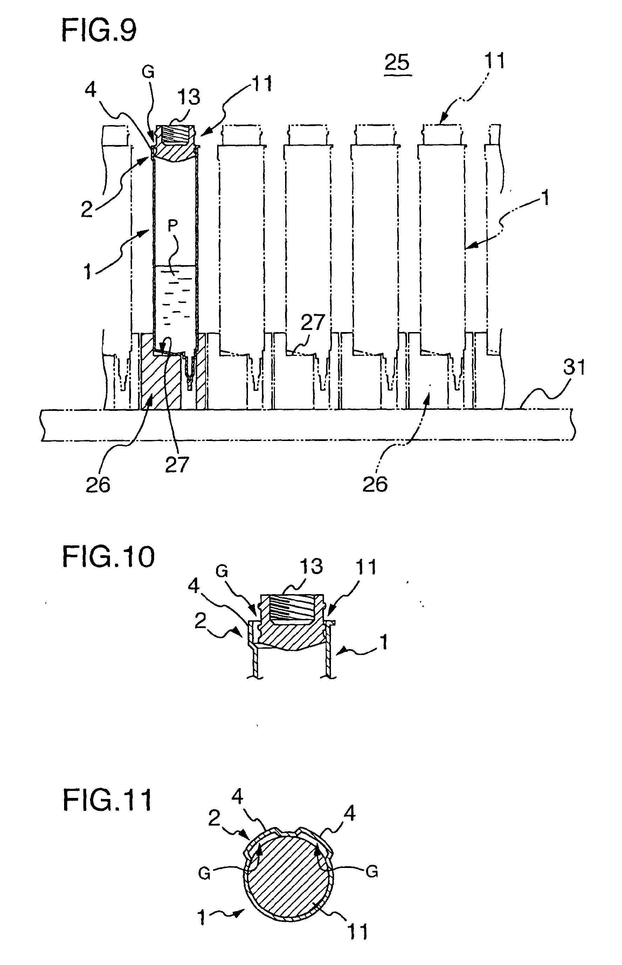

Fig. 6 shows the elastic stopper 11 to be inserted with hermetic seal into the syringe outer tube 1, three sealing circumferential protrusions 12 contacting an inner peripheral surface of the syringe outer tube 1 are formed on an outer peripheral surface thereof, and a female screw 13 is arranged on a bottom thereof so that a male screw 22 projecting on a front end of the plunger 21 is screwed into the female screw 13 to joining the elastic stopper 11 and the plunger 21 (refer to Fig. 1). When the elastic stopper 11 is fitted into the base portion 2 of the syringe outer tube 1 by a small depth (refer to Figs. 9 and 10), clearances G for discharging the air are formed between the recesses 4 and the elastic stopper 11 (refer to Fig. 11).

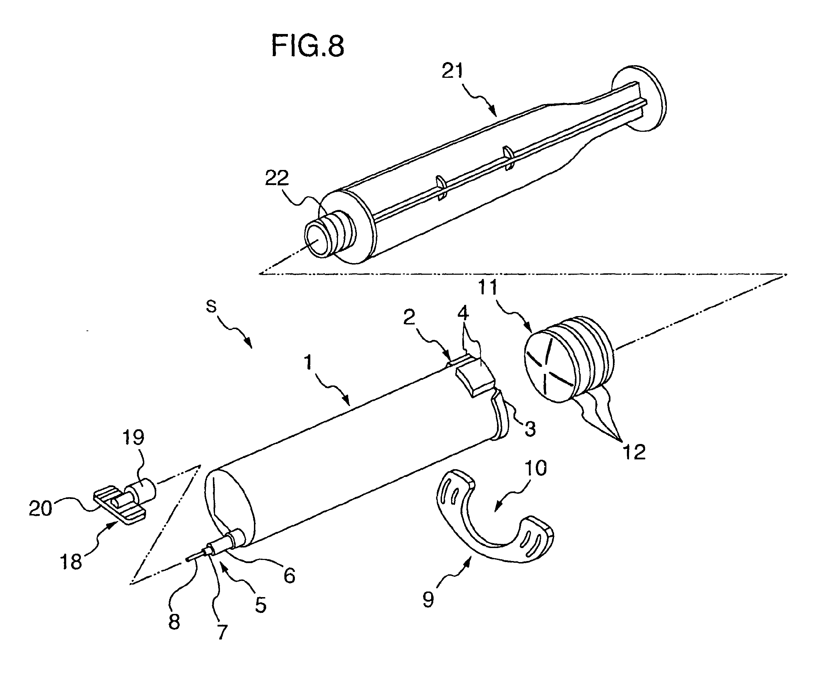

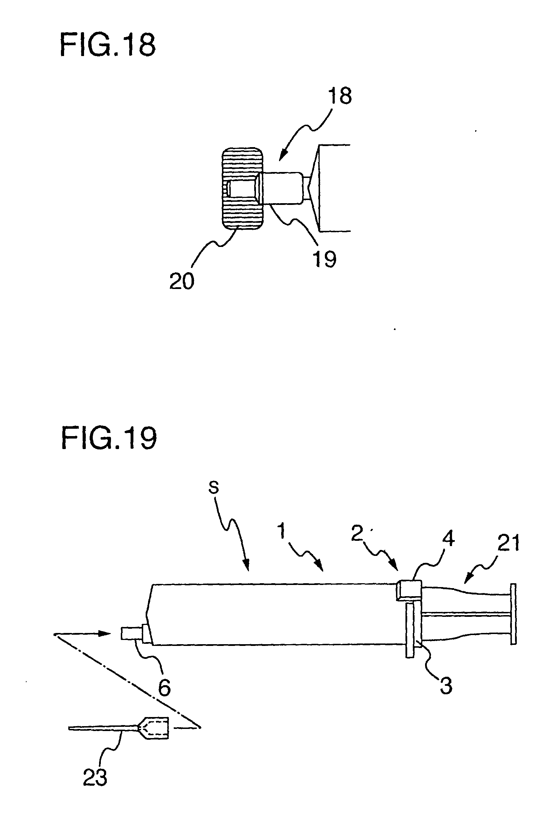

Fig. 7 shows a cutting tool 18 operating also as a cap, a grasping portion 20 is monolithically formed on a back portion of a tube-shaped portion 19 to be fitted onto the projection 5 for the discharge hole of the syringe outer tube 1, the tube-shaped portion 19 is fitted onto the outer surface of the projection 5 for the discharge hole to protect the projection 5 for the discharge hole when being stored (refer to Figs. 8 and 18), and the grasping portion 20 is grasped to bend the cut groove 7 to be cut off while moving back the tube-shaped portion 19 slightly from the fitting position, so that the base portion 6 of the projection 5 for the discharge hole is opened to become usable by removing the cut portion 8.

Fig. 8 is an oblique projection view of a disassembled syringe S formed by the syringe outer tube 1, external flange 9, elastic stopper 11 and plunger 21. The syringe outer tube 1, external flange 9 and plunger 21 are made of a plastic material such as polyethylene (PE), polypropylene (PP), cyclic olefin copolymer (COC), silica deposited plastic or the like. The polypropylene (PP) is light and cheap, and has a good machinability (moldability), no effluence and good chemical resistance so that the above medicine package set can be provided cheaply, and PP is recyclable while treatment thereof easy so that it is advantageous in environmental problem.

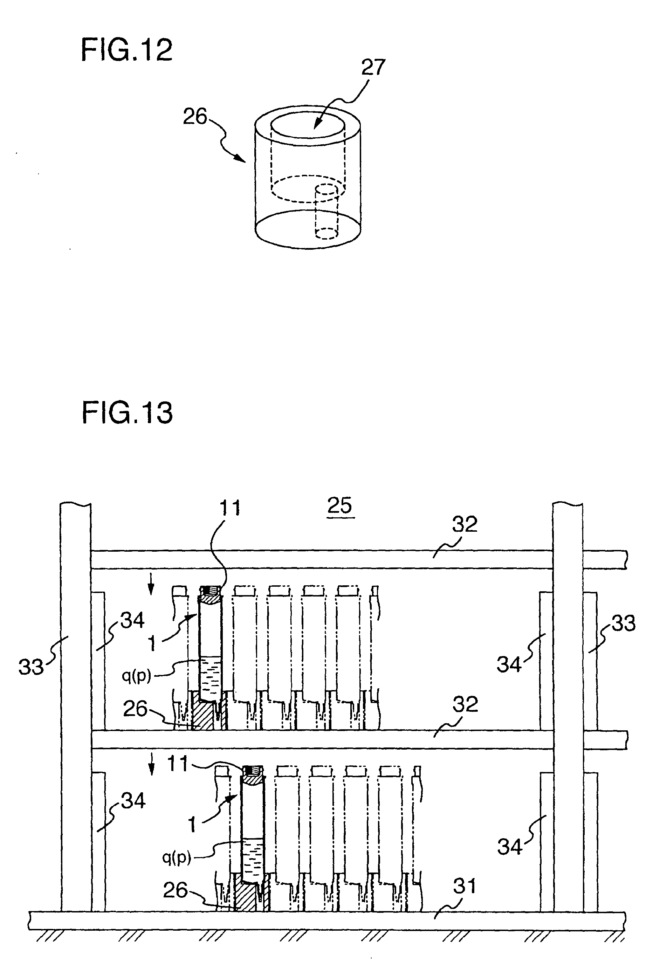

Fig. 9 shows a condition in which each of the syringe outer tubes 1 is charged with a medicine liquid p of a unit amount (10-300mg at freeze-dried condition) to be used, and the elastic stoppers 11 are inserted in to the base portions 2 at a small depth while being vertically aligned in a freeze-drying room 25. Each of the syringe outer tubes 1 is stably supported by fitting the front end thereof into a fitting recess 27 of a supporting member 26 (refer to Fig. 12). In this aligned condition, a distance between the syringe outer tubes 1 is set small by removing the outer flange 9 so that the syringe outer tubes 1 of larger total number are contained in the limited freeze-drying room 25.

A reason as to why the front end of the elastic stopper 11 is inserted into the base portion 2 of the syringe outer tube 1 at the small depth in the freeze-drying room 25 as described above (refer to Figs. 9 and 10) is for forming clearances G relative to clearance forming recesses 4 to allow a gas to be discharged from an inside of the syringe outer tube 1 to an outside during the freeze-drying under a decreased pressure. Incidentally, a number of the recesses 4 may be one, or not less than three.

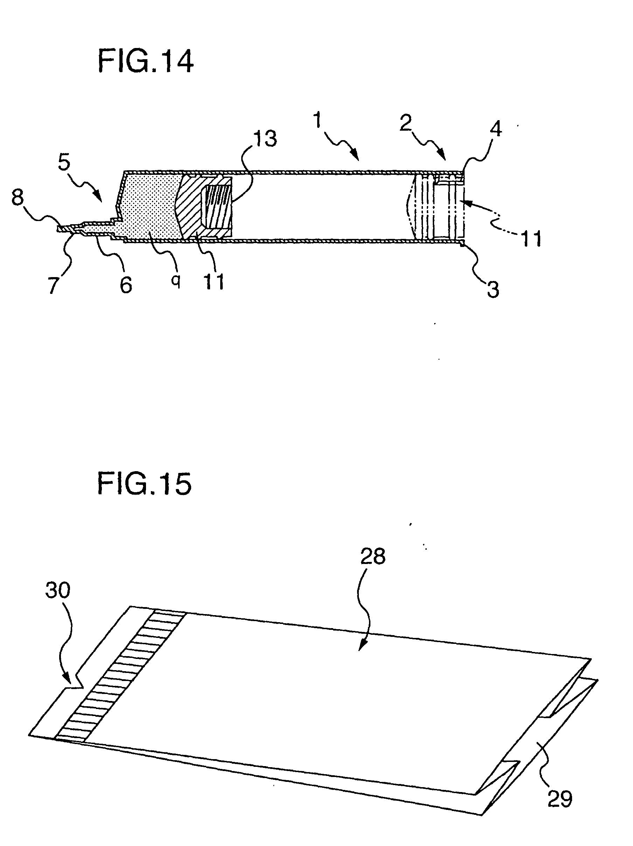

Fig. 13 is an explanatory drawing of a device for closing the syringe outer tube 1 by the elastic stopper 11 when a freeze-drying of the medicine liquid p is completed in the freeze-drying room 25, 31 denotes a stationary floor member, 32 denotes a movable plate member sliding vertically, 33 denotes a guide rail for guiding the movable plate member 32 vertically, 34 denotes a stopper, when the freeze-drying of the medicine liquid p is completed in each of the syringe outer tubes 1 held at the aligned condition, the movable plate member 32 is moved downward to contact a bottom portion of each of the elastic stopper 11, and the bottom portion is pressed down to a position of an end of the base portion 2 of the syringe outer tube 1 (refer to a two-dot chain line in Fig. 14) so that each of the syringe outer tubes 1 is closed by the elastic stopper 11.

Just after a door of the freeze-drying room 25 is opened, the elastic stopper 11 closing each of the syringe outer tubes 1 under the decreased pressure as described above is pressed downward toward the front end (bottom portion) of the syringe outer tube 1 by the atmospheric pressure to hermetically seal the freeze-dried medicine q (refer to Fig. 14). The atmospheric pressure is applied to the bottom of the elastic stopper 11 to press the elastic stopper 11 downward during the sealing process by the atmospheric pressure, so that the inside of the syringe outer tube 1 is hermetically sealed by the elastic stopper 11 to prevent the medicine q from contacting the atmospheric air and being contaminated.

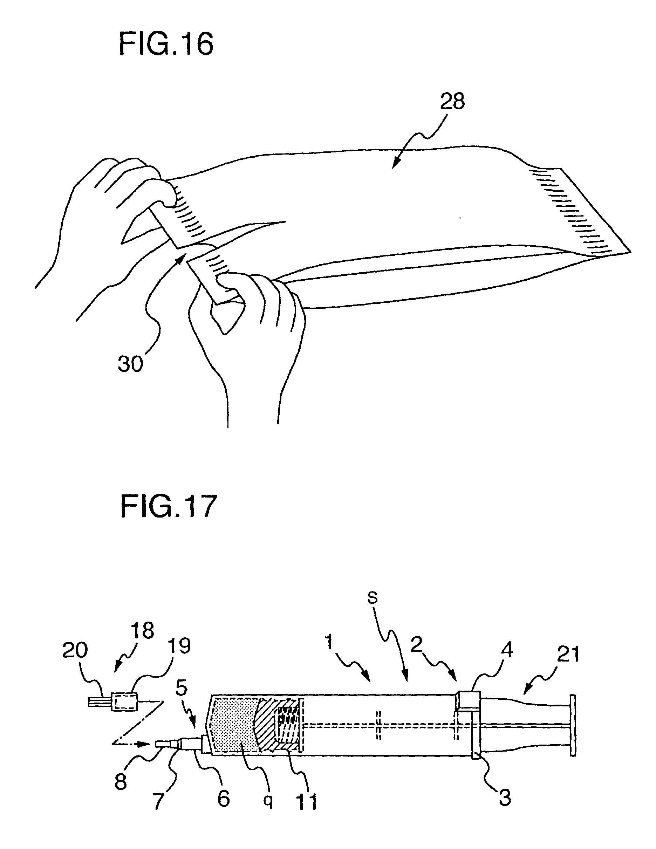

Subsequently, a male screw 22 at a front end of the plunger 21 is screwed into a female screw 13 of the elastic stopper 11 to integrate the elastic stopper 11 and the plunger 21 (refer to Fig. 1), and a cutting tool 18 is fitted on the projection 5 for the discharge hole (refer to Figs. 17 and 18), this is contained together with the injection needle 23 (refer to Fig. 19) and outer flange 9 (refer to Fig. 5) by a separately prepared sealing bag 28 made of a flanged aluminum or silica deposited film (refer to Fig. 15), and an opening 29 thereof is sealed so that the syringe outer tube 1 with the hermetically sealed medicine q therein is assembled with the other attachments to form a set to be transferred in an aseptic condition from a factory through a distribution system to each hospital and stored therein.

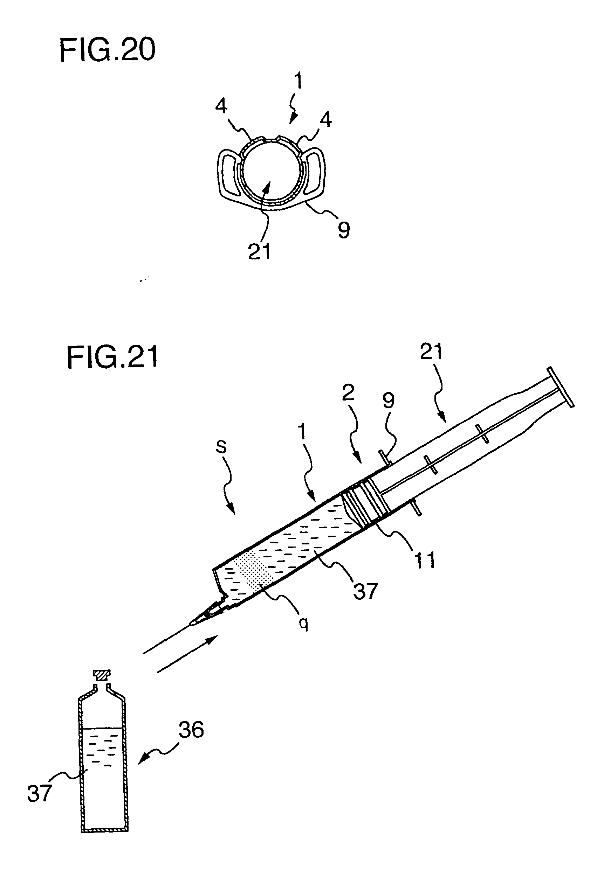

When this medicine package set F is used, a notch portion 30 (refer to Fig. 16) formed on a bottom portion of the sealing bag 28 is cut off to take out the contained matters, at first, the cutting tool 18 fitted on the projection 5 for the discharge hole is slightly moved backward from the fitting position shown in Fig. 18 and bent to cut off the cut groove 7 (refer to Fig. 4) and remove the cut portion 8, and the injection needle 23 is mounted on the base portion 6 while the outer flange 9 is mounted on the base portion 2 of the syringe outer tube 1 (refer to Fig. 19 and 20).

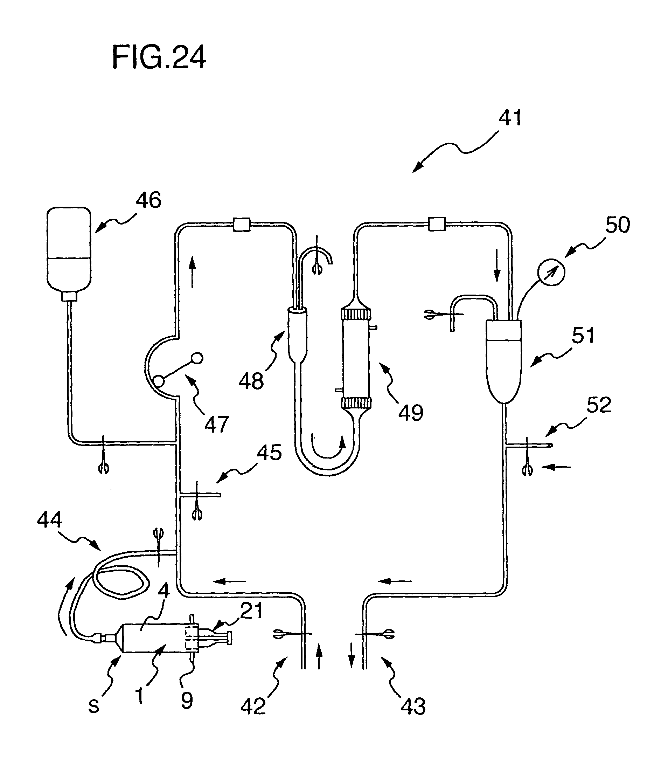

Subsequently, a separately prepared dissolution liquid 37 (for example, 5%-glucose injection) in a container 36 is injected into the syringe outer tube 1 to dissolve the medicine q (refer to Fig. 21), and immediately this is set on the syringe mounting portion 16 of the dialyser (or syringe pump) 15 (refer to Figs. 22 and 23) to start an injection of the medicine q into an extracorporeal circulation path 41 (refer to Fig. 24). Therefore, a complicated preparation in the prior art is not necessary and the setting operation is made significantly easy.

The syringe mounting portion 16 of the dialyser 15 (refer to Figs. 22 and 23) has a pair of grooves 161 for fitting the syringe outer tubes 1 therein, an engage groove 162 for engaging the outer flanges 9, a fixing member 163 for pressing the syringe outer tubes 1 to be fixed, and a moving arm 164 for pressing the base portion of the plunger 21 to move. The moving arm 164 is moved in a guide groove 165 by a driving source in a body of the dialyser 15 to supply the dissolved medicine q in the set syringe outer tube 1 by a discharged amount per a predetermined time period (constant discharging speed).

As described above, the syringe outer tube 1 can be set on the device just after the dissolution liquid 37 is injected into the syringe outer tube 1 taken out of the sealing bag 28 to dissolve the medicine q, and an operation for setting is made significantly easy, so that an operating efficiency is remarkably improved and a degree of contamination of the medicine q in this time period is significantly decreased in comparison with the prior art.

Fig. 24 shows a circuit (external circulation path 41) during dialyzing, 42 is an artery side circuit, 43 is a vein side circuit, 44 is a heparin circuit, 45 is a bypass circuit, 46 is a physiological saline solution liquid, 47 is a blood pump, 48 is an air trap, 49 is a dialyser, 50 is a pressure meter, 51 is an air trap, 52 is a bypass circuit, the syringe outer tube 1 is connected to the heparin circuit 44 so that the medicine q thereof inhibits an activity of a protease in the external circulation path 41 to prevent effectively a blood coagulation.

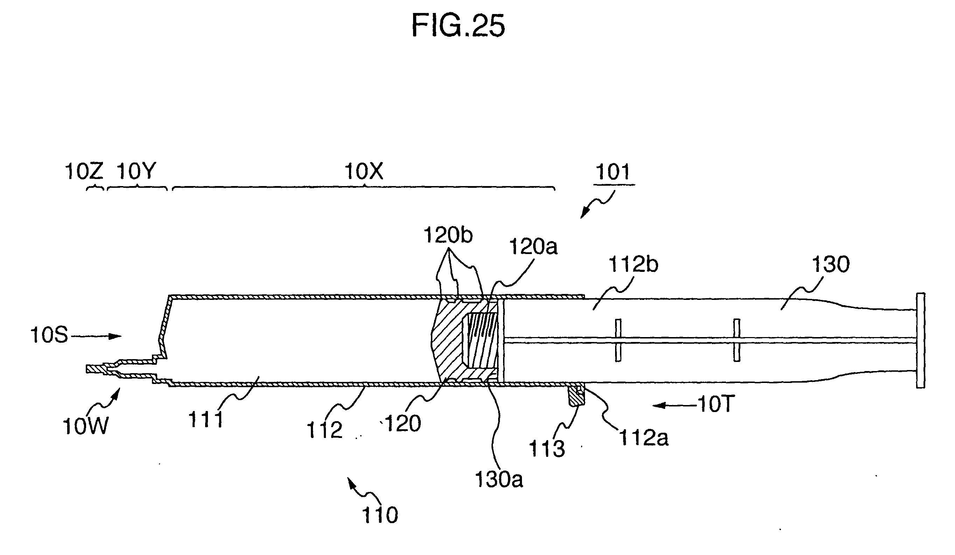

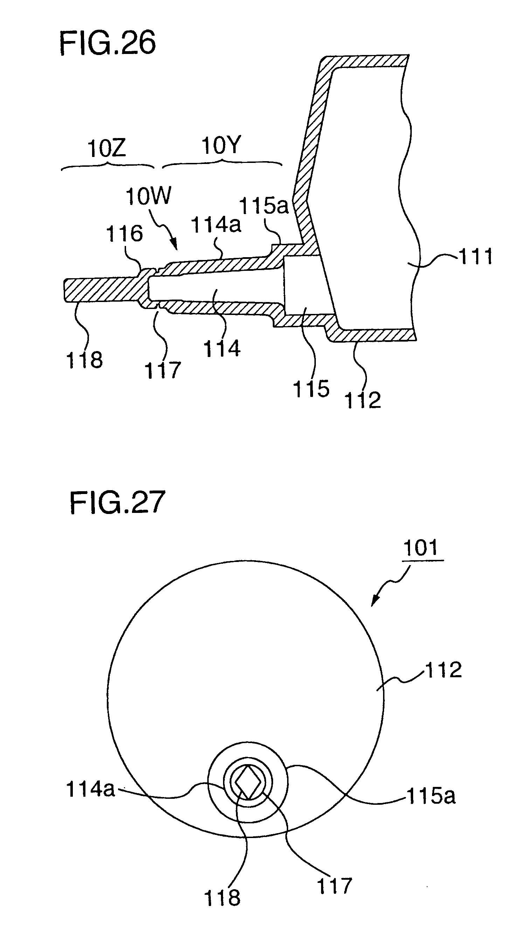

Another preferred embodiment is described below. Fig. 25 is a view showing a whole of a structure of a syringe 101, Fig. 26 is an enlarged cross sectional view of a discharging portion, and Fig. 27 is a front view (view seen from a direction of an arrow 10S in Fig. 25) of the syringe shown in Fig. 25.

The syringe 101 has mainly a syringe body 110 and a plunger 130, and the syringe body 110 is divided to a charged portion 10X, a discharging portion 10Y and a jig mounting portion 10Z. The charged portion 10X has a charged tubular space 111 to be charged with the medicine, an outer tube 112 forming the 111, and a flange mounting portion 112c for mounting a flange 113. The outer tube 112 is cylindrical or substantially cylindrical, has the discharging portion 10Y at an end thereof, and an inlet portion 112a at another end for inserting the medicine and the plunger 130. The end of the outer tube 112 with the discharging portion 10Y has a frustum of cone shape.

A material forming the syringe 101 is preferably polyethylene (PE), polypropylene (PP), cyclic olefin copolymer (COC), silica deposited plastic or the like. By using the resin, the charged portion 10X, discharging portion 10Y and jig mounting portion 10Z are formed monolithically.

As shown intelligibly in Fig. 26, the discharging portion 10Y extends from the end of the outer tube 112 as a needle, and a first discharge path 114 and a second discharge path 115 are formed in an interconnection condition. The first discharge path 114 is formed by a first path forming tube 114a, and the second discharge path 115 is formed by a second path forming tube 115a, respectively. As a size of the discharge path, for example, the first discharge path 114 is φ2.0-2.4 mm of tubular shape, and the second discharge path 115 is φ4.2 mm of tubular shape.

As shown in Fig. 26, a front end of the first discharge path 114 is closed, so that the medicine inserted in the charged tubular space 111 is not discharged in this condition. A weak portion 10W is formed at a front end of the first path forming tube 114a, and is composed of a notch forming tube 116 with an outer diameter smaller than the first path forming tube 114a, and a notch 117 formed on an outer periphery thereof. An outer diameter of the notch forming tube 116 is about φ2.8 mm, and a notch 117 is formed as a V-cross-sectional shaped groove of depth of about 0.15 mm. By breaking the notch 117, the first discharge path 114 is opened. A position of the notch 117 is at the front end of the first discharge path 114 or adjacent to the front end.

Incidentally, as a concrete structure of the weak portion 10W, a gradually decreasing thickness structure other than the notch 117 may be used. The notch 117 may be a slit-shaped groove, a concave groove or the other various shape.

A jig mounting column 118 operating as a jig mounting portion Z is formed at a forward end of the . notch forming tube 116. The jig mounting column 118 is formed as a rhomb shaped cross section column, and a size of the rhomb is, for example, of diagonal length of 2.0 mm X 2.8 mm. The cross sectional shape of the jig mounting column 118 may be rectangular, for example, square or oblong.

As shown in Fig. 27, the first path forming tube 114a and so forth are arranged at a position eccentric from a center of the outer tube 112.

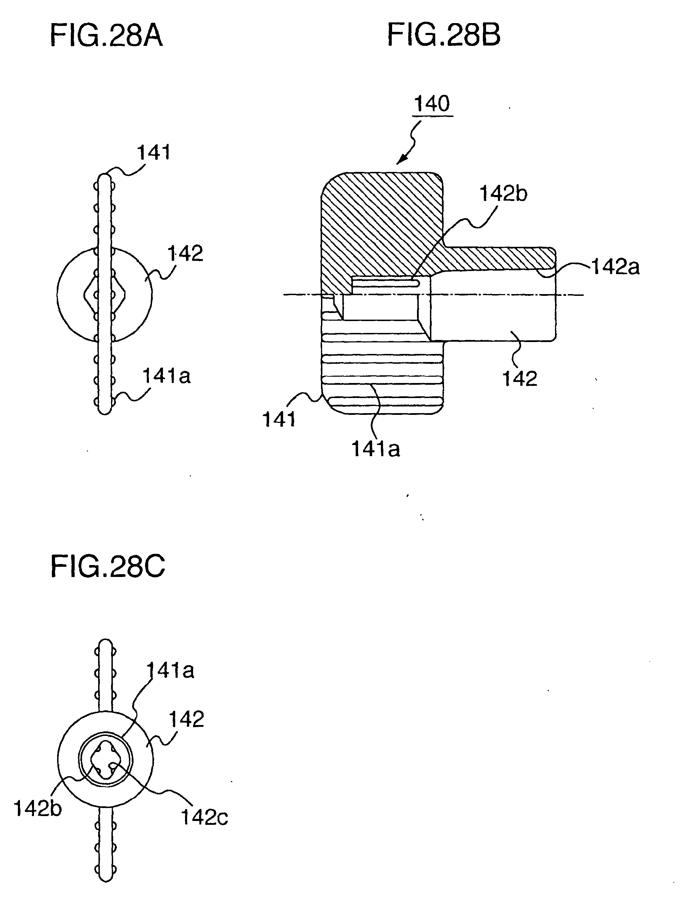

As a next, a structure of a jig mounted on the jig mounting portion 10Z is explained on Fig. 28. Fig. 28(a) is a front view, (b) is a side view whose upper side is a cross section and whose lower side shows an exterior appearance, and (c) is a back view, respectively. A jig 140 includes a pinching portion 141 to be operated by fingers, and a body portion 142. The pinching portion 141 is flat plate shaped, and has many projection 141a for easy pinching on an outer surface. The body portion 142 has a guide hole 142a, a rhomb shaped hole 142b into which the jig mounting column 118 is inserted, and four small projections 142c on an inner surface of the rhomb shaped hole 142b. The jig 140 is made of, for example, polyethylene (PE), polypropylene (PP) or the like.

Briefly explaining a method of using the jig 140, the jig mounting column 118 of the jig mounting portion 10Z of the syringe is inserted into the rhomb shaped hole 142b of the jig 140 and rotated on an axis of the syringe 101 by pinching the pinching portion 141 with fingers, so that the notch 117 of the weak portion 10W is broken to open the first discharge path 114.

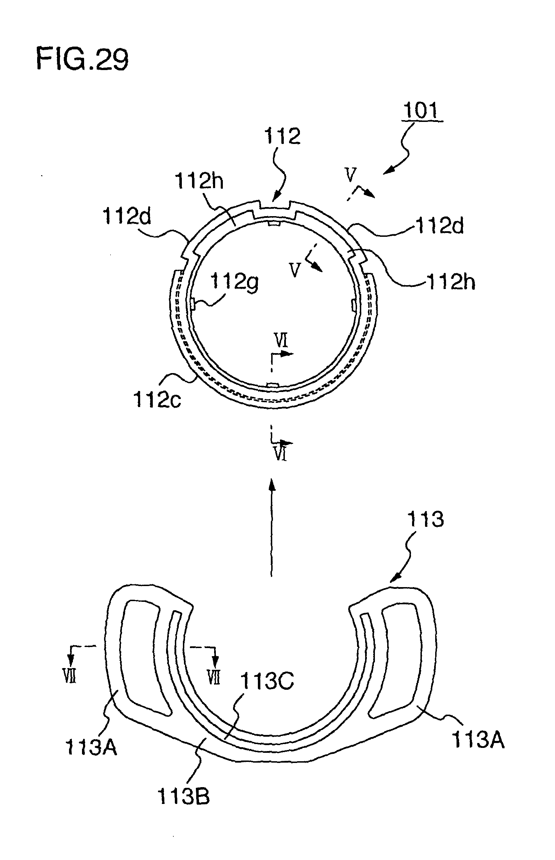

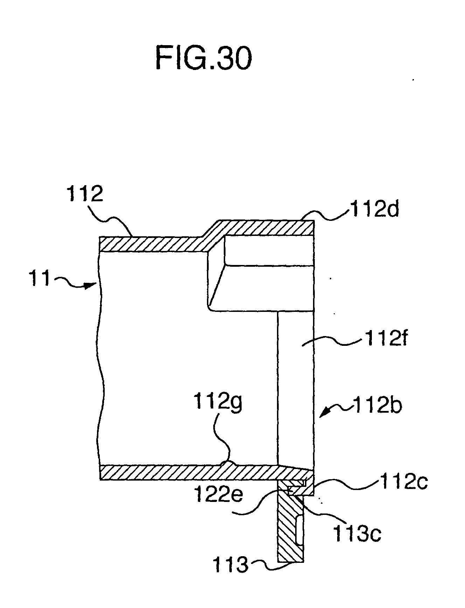

On Figs. 29 and 30, structures of the outer tube 112 around the inlet portion 112b and the flange 113 are explained. Fig. 29 is a view as seen from an arrow 10T in Fig. 25 (the plunger 130 is eliminated) while the flange 113 is removed from the outer tube 112 of the syringe 101 for easy understanding. Fig. 30 is an enlarged cross sectional view of the inlet portion 112b, an upper half thereof shows V-V cross section of Fig. 29, a lower half thereof shows VI-VI cross section of Fig. 29, and a portion showing the flange 13 shows VII-VII cross section of Fig. 29, respectively.

As shown in Figs. 29 and 30, the inlet portion 112b has a flange mounting portion 112c for mounting the flange 113, and enlarged diameter portions 112d at two circumferential positions. A mounting projection 112e is formed circumferentially on the flange mounting portion 112c. The enlarged diameter portions 112d have inner diameters slightly larger than an inner diameter of the outer tube 112. Further, a tapered surface 112f is formed on the inlet portion 112b for easy insertion of a gasket 120, and ball-shaped projections 112g are formed at four circumferential positions deeper than the tapered surface 112f.

As shown in Fig. 29, the flange 113 has a pair of left and right jaw portions 113a, a connection portion 113b connecting the jaw portions 113a, and a circumferential groove 113c on which a mounting projection 112g is fitted. Fig. 30 shows a condition in which the flange 113 is mounted.

In Fig. 25, the gasket 120 is attached to the front end of the plunger 130. The gasket 120 is made of preferably a rubber, and has the female screw 120a to be screwed onto the male screw 130a formed on the front end of the plunger 130, and annular projections 120b on an outer surface. As shown in Fig. 25, the charged space 111 is hermetically sealed from the outside when the gasket 120 is pressed into the charged space 111 by a certain degree, but the charged space 111 and the outside communicate with each other through clearances 112h (refer to Fig. 29) formed on the enlarged diameter portions 112d when the gasket 120 is mounted on the inlet portion 112b.

As a next, a method of using the syringe 101 of the embodiment of the invention is explained. Any medicine which can be dissolved by a water and freeze-dried is usable as the medicine with which the syringe 101 is charged.

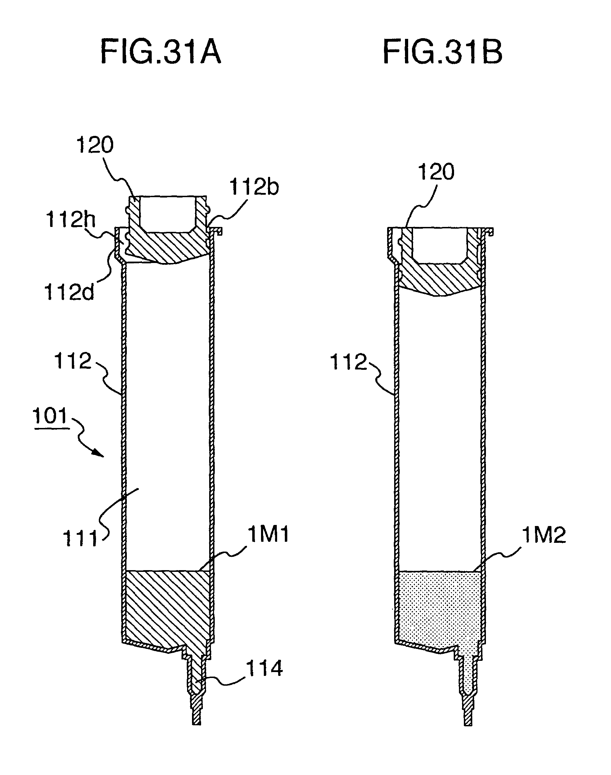

At first, the syringe 101 is vertically set as shown in Fig. 31, and a medicine liquid 1M1 of aqueous solution of the medicine is inserted through the inlet potion 112b of the syringe 101. The syringe 101 is kept vertically while the first discharge path 114 is closed, so that the medicine liquid 1M1 does not leak. Subsequently, as shown in Fig. 31(a), the gasket 120 is mounted on the inlet portion 112b. In this condition, the charged space 111 and the outside communicate with each other through the clearances 112h.

Subsequently, many of the syringes 101 charged with the medicine liquid 1M1 is set in the well known freeze-drying device (not shown). Since the flanges 113 are removed in this condition, the syringes 101 can be arranged in a space therefor. In the freeze-drying device, the medicine liquid 1M1 is frozen, a vacuuming is performed, and a drying is performed to form a powder medicine 1M2. In spite of the gasket 120, a vaporized moisture can flow out through the clearances 112h. And, as shown in Fig. 31(b), the gasket 120 is pressed down after the freeze-drying process to a position at which an upper surface reaches a height of an upper end surface of the outer tube 112. By this operation, the charged space 111 is hermetically sealed. Subsequently, a room in which the syringes 101 are arranged is returned from a vacuumed pressure to the atmospheric pressure, the gasket 120 is pressed into the inside of the outer tube 112 by a difference between the pressure in the charged space 111 and the atmospheric pressure. In this condition, the medicine 1M2 can be stored in the syringe 101.

A case in which the medicine 1M2 is used is explained below. The before mentioned jig 140 is mounted on the jig mounting portion 10Z of the syringe 101, and the pinching portion 141 of the jig 140 is rotated by fingers. By this operation, the closed condition of the first discharge path 114 is released, so that the powdered medicine 1M2 is dissolved by mounting the injection needle onto the discharging portion 10Y of the syringe 101 and injecting the medicine dissolution liquid of a predetermined amount into the charged space 111. For example, this syringe 101 is mounted onto the syringe pump to feed the medicine of a predetermined amount per hour.

The syringe 101 is set on the syringe pump, to press the plunger 130 to feed out the medicine of the predetermined amount. Incidentally, the mounted flange 113 is set on a groove of the syringe pump to position the syringe 101 on the syringe pump.

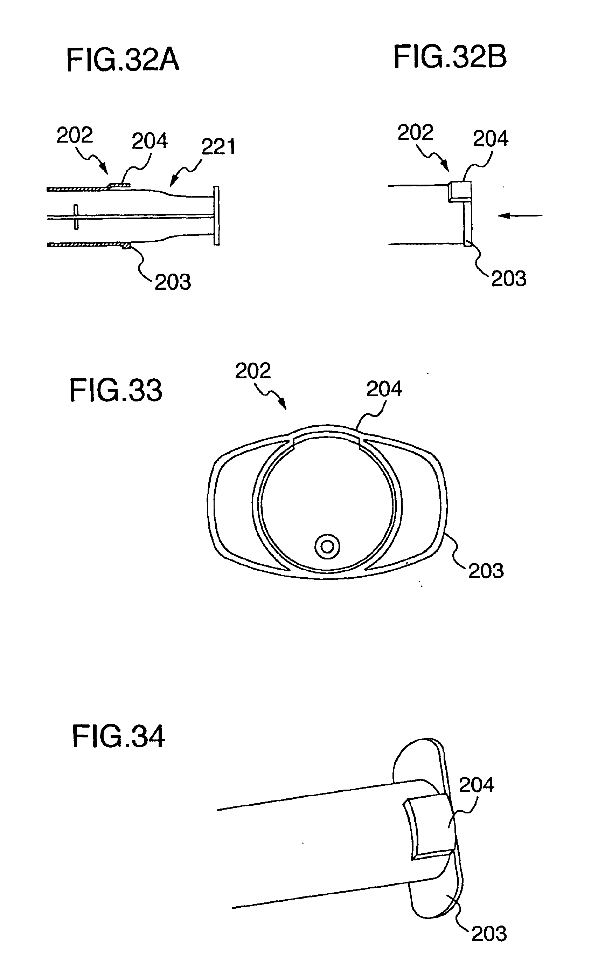

As shown in Figs. 32-34, the detachable outer flange 9 and flange 113 may be replaced by a fixed flange 203 extending radially outward from the syringe 202. The syringe 202 has a clearance 204 extending radially distantly from the plunger 221 so that the moisture is discharged out of the syringe through the clearance portion 204 when freeze-drying the medicine while the stopper is mounted on the syringe.

## Claims

1. A container for a medicine, comprising,
a chamber for containing the medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, wherein by breaking at least a portion of the breakable part to separate the closing part from the container, the medicine is allowed to be discharged to the outside of the container through the broken breakable part while the medicine is prevented from passing the inlet passage.

2. A container according to claim 1, wherein the body, the closing part and the breakable part are monolithic.

3. A container according to claim 1, wherein the body, the closing part and the breakable part are monolithically formed by a synthetic resin.

4. A container according to claim 1, wherein a cross-sectional area of the breakable part along an imaginary plane perpendicular to a flow direction of the medicine discharged to the outside of the container is significantly smaller than a cross-sectional area of the body along the imaginary plane perpendicular to the flow direction of the medicine discharged to the outside of the container and a cross-sectional area of the closing part along the imaginary plane perpendicular to the flow direction of the medicine discharged to the outside of the container.

5. A container according to claim 1, further comprising a stopper slideable in the inlet passage while sealing hermetically the inlet passage.

6. A container according to claim 1, further comprising the medicine of dry condition contained by the chamber.

7. A container according to claim 1, further comprising a stopper guided by the inlet passage to slide therein, wherein the inlet passage includes a first area which is relatively far away from the breakable part and forms a clearance between the inlet passage and the stopper to allow a gaseous communication between the chamber and the outside of the container, and a second area which is relatively near by the breakable part and is hermetically sealed by the stopper.

8. A container according to claim 1, further comprising a stopper slideable in the inlet passage while sealing hermetically the inlet passage, and an injection needle, wherein the medicine is discharged to the outside of the container through the breakable part and the injection needle when the stopper moves in the inlet passage toward the breakable part.

9. A container according to claim 1, further comprising a flange extending perpendicularly to a longitudinal axis of the inlet passage, wherein a distance between the flange and the breakable part in a longitudinal direction of the inlet passage is prevented from becoming not less than a predetermined distance.

10. A container according to claim 9, wherein the body includes a protrusion extending perpendicularly to a longitudinal axis of the inlet passage, wherein the flange discrete relative to the body contacts the protrusion to prevent the distance between the flange and the breakable part in the longitudinal direction of the inlet passage from becoming not less than the predetermined distance.

11. A container according to claim 1, further comprising a cover covering the closing part, wherein the cover extends over the breakable part to fit onto the body.

12. A container according to claim 11, wherein the cover is prevented from contacting the breakable part when fitting onto the body.

13. A container according to claim 1, wherein the inlet passage, closing part and breakable part are substantially aligned along an axis parallel to a longitudinal axis of the inlet passage.

14. A method for containing a medicine into a container, comprising the steps of:
preparing the medicine container comprising a chamber for containing the medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, wherein the medicine is allowed to be discharged to the outside of the container through the broken breakable part when at least a portion of the breakable part is broken to separate the closing part from the container,
feeding the medicine into the chamber,
positioning a stopper at a first area of the inlet passage relatively far away from the breakable part so that a clearance between the inlet passage and the stopper is formed to allow a gaseous communication between the chamber and the outside of the container,
drying the medicine by discharging a moisture from the chamber through the clearance to the outside of the container, and
positioning the stopper at a second area of the inlet passage relatively near by the breakable part so that the inlet passage is hermetically sealed by the stopper.

15. A method according to claim 14, wherein a gaseous pressure in the chamber is less than an atmospheric pressure when the stopper is being positioned at the second area relatively near by the breakable part, and the container is arranged in a gaseous environment of the atmospheric pressure after the stopper is positioned at the second area relatively near by the breakable part.

16. A method according to claim 14, wherein a temperature in the chamber is less than 50°C when the stopper is being positioned at the second area relatively near by the breakable part, and the container is arranged in an environment of room temperature after the stopper is positioned at the second area relatively near by the breakable part.

## Amended claims

### Amended claims under Art. 19.1 PCT

1. A container for a medicine, comprising,
a chamber for containing the medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, wherein by breaking at least a portion of the breakable part to separate the closing part from the container, the medicine is allowed to be discharged to the outside of the container through the broken breakable part while the medicine is prevented from passing the inlet passage.

2. A container according to claim 1, wherein the body, the closing part and the breakable part are monolithic.

3. A container according to claim 1, wherein the body, the closing part and the breakable part are monolithically formed by a synthetic resin.

4. A container according to claim 1, wherein a cross-sectional area of the breakable part along an imaginary plane perpendicular to a flow direction of the medicine discharged to the outside of the container is significantly smaller than a cross-sectional area of they body along the imaginary plane perpendicular to the flow direction of the medicine discharged to the outside of the container and a cross-sectional area of the closing part along the imaginary plane perpendicular to the flow direction of the medicine discharged to the outside of the container.

5. A container according to claim 1, further comprising a stopper slideable in the inlet passage while sealing hermetically the inlet passage.

6. A container according to claim 1, further comprising the medicine of dry condition contained by the chamber.

7. A container according to claim 1, further comprising a stopper guided by the inlet passage to slide therein, wherein the inlet passage includes a first area which is relatively far away from the breakable part and forms a clearance between the inlet passage and the stopper to allow a gaseous communication between the chamber and the outside of the container, and a second area which is relatively near by the breakable part and is hermetically sealed by the stopper.

8. A container according to claim 1, further comprising a stopper slideable in the inlet passage while sealing hermetically the inlet passage, and an injection needle, wherein the medicine is discharged to the outside of the container through the breakable part and the injection needle when the stopper moves in the inlet passage toward the breakable part.

9. A container according to claim 1, further comprising a flange extending perpendicularly to a longitudinal axis of the inlet passage, wherein a distance between the flange and the breakable part in a longitudinal direction of the inlet passage is prevented from becoming not less than a predetermined distance.

10. A container according to claim 9, wherein the body includes a protrusion extending perpendicularly to a longitudinal axis of the inlet passage, wherein the flange discrete relative to the body contacts the protrusion to prevent the distance between the flange and the breakable part in the longitudinal direction of the inlet passage from becoming not less than the predetermined distance.

11. A container according to claim 1, further comprising a cover covering the closing part, wherein the cover extends over the breakable part to fit onto the body.

12. A container according to claim 11, wherein the cover is prevented from contacting the breakable part when fitting onto the body.

13. A container according to claim 1, wherein the inlet passage, closing part and breakable part are substantially aligned along an axis parallel to a longitudinal axis of the inlet passage.

14. A method for containing a medicine into a container, comprising the steps of :
preparing the medicine container comprising a chamber for containing the medicine in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, and a breakable part between the body and the closing part, wherein the medicine is allowed to be discharged to the outside of the container through the broken breakable part when at least a portion of the breakable part is broken to separate the closing part from the container,
feeding the medicine into the chamber,
positioning a stopper at a first area of the inlet passage relatively far away from the breakable part so that a clearance between the inlet passage and the stopper is formed to allow a gaseous communication between the chamber and the outside of the container,
drying the medicine by discharging a moisture from the chamber through the clearance to the outside of the container, and
positioning the stopper at a second area of the inlet passage relatively near by the breakable part so that the inlet passage is hermetically sealed by the stopper.

15. A method according to claim 14, wherein a gaseous pressure in the chamber is less than an atmospheric pressure when the stopper is being positioned at the second area relatively near by the breakable part, and the container is arranged in a gaseous environment of the atmospheric pressure after the stopper is positioned at the second area relatively near by the breakable part.

16. A method according to claim 14, wherein a temperature in the chamber is less than 50°C when the stopper is being positioned at the second area relatively near by the breakable part, and the container is arranged in an environment of room temperature after the stopper is positioned at the second area relatively near by the breakable part.

17. (added) A container containing a medicine, comprising,
a chamber for containing the medicine of dry condition in at least a portion of the chamber, an inlet passage extending between the chamber and an outside of the container to allow the medicine to be supplied into the chamber through the inlet passage, a closing part forming a part of the chamber, a body forming the inlet passage and another part of the chamber, a breakable part between the body and the closing part, and a stopper slideable in the inlet passage while hermetically sealing the inlet passage, wherein by breaking at least a portion of the breakable part to separate the closing part from the container, a dissolution liquid is inserted into the chamber to dissolve the medicine of dry condition.

18. (added) A container according to claim 17, wherein the body, closing part and breakable part are monolithic.

19. (added) A container according to claim 17, wherein the body, closing part and breakable part are monolithically formed of a synthetic resin.

20. (added) A container according to claim 17, wherein the stopper includes a female screw engageable with a male screw of a front end of a plunger.

21. (added) A container according to claim 17, wherein the stopper is monolithically formed with a plunger.

22. added) A container according to claim 17, wherein the container is contained by a sealing bag whose opening is sealed.

23. (added) A container according to claim 22, wherein the sealing bag is made of an aluminum deposited film member or a silica deposited film member.

24. (added) A container according to claim 17, further comprising a cover covering the closing part, wherein the cover extends over the breakable part to fit onto the body part.

25. (added) A container according to claim 24, wherein the cover is prevented from contacting the breakable part when fitting onto the body part.
Explanation under Article 19(1)
Attached substitute pages 16, 17, 18 and 18/1 of claims are filed to add claims 17-25.
Claim 17 is supported by recitations of claims 1, 5 and 6 and description on lines 15-29, page 10 of the specification. Claims 18 and 19 are supported by claims 2 and 3, claim 20 is supported by description on lines 13-14, page 10 of the specification, claim 21 is supported by description on lines 14-15, page 10 of the specification, claims 22 and 23 are supported by description on lines 17-19, page 10 of the specification, and claims 24 and 25 are supported by claims 11 and 12 respectively.
